(19) Europäisches Patentamt
European Patent Office
Office européen des brevets

(11) Veröffentlichungsnummer: **0 572 863 A1**

(12) # EUROPÄISCHE PATENTANMELDUNG

(21) Anmeldenummer: **93108129.3**

(22) Anmeldetag: **19.05.93**

(51) Int. Cl.⁵: **C07D 487/04**, A61K 31/495, //(C07D487/04,241:00,209:00)

(30) Priorität: **05.06.92 CH 1819/92**
**29.04.93 CH 1307/93**

(43) Veröffentlichungstag der Anmeldung:
**08.12.93 Patentblatt 93/49**

(84) Benannte Vertragsstaaten:
**AT BE CH DE DK ES FR GB GR IE IT LI LU MC NL PT SE**

(71) Anmelder: **F. HOFFMANN-LA ROCHE AG**
**Postfach 3255**
**CH-4002 Basel(CH)**

(72) Erfinder: **Bös, Michael**
**8A Marktgasse**
**CH-4310 Rheinfelden(CH)**

(74) Vertreter: **Bertschinger, Christoph, Dr. et al**
**Grenzacherstrasse 124**
**P.O. Box 3255**
**CH-4002 Basel (CH)**

(54) **ZNS Pyrazinoindole.**

(57) Die neuen Pyrazinoindole der allgemeinen Formel

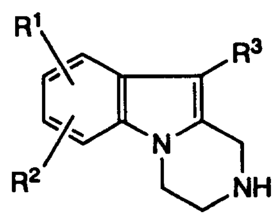

I

worin $R^1$ Wasserstoff, Halogen, Trifluormethyl, niederes Alkyl, Hydroxy oder niederes Alkoxy, $R^2$ Wasserstoff oder Halogen und $R^3$ Wasserstoff, niederes Alkoxy oder niederes Alkylthio bedeuten, mit der Massgabe, dass $R^3$ nur dann Wasserstoff bedeuten kann, wenn $R^1$ und $R^2$ beide von Wasserstoff verschieden sind
und pharmazeutisch annehmbare Säureadditionssalze der Verbindungen der Formel I eignen sich zur Behandlung oder Verhütung von zentralnervösen Störungen, wie Depressionen, bipolare Störungen, Angstzustände, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

EP 0 572 863 A1

Die vorliegende Erfindung betrifft Pyrazinoindole. Im speziellen betrifft sie Verbindungen der allgemeinen Formel

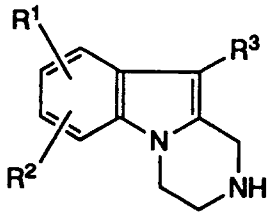

I

worin $R^1$ Wasserstoff, Halogen, Trifluormethyl, niederes Alkyl, Hydroxy oder niederes Alkoxy, $R^2$ Wasserstoff oder Halogen und $R^3$ Wasserstoff, niederes Alkoxy oder niederes Alkylthio bedeuten, mit der Massgabe, dass $R^3$ nur dann Wasserstoff bedeuten kann, wenn $R^1$ und $R^2$ beide von Wasserstoff verschieden sind,
und pharmazeutisch annehmbare Säureadditionssalze der Verbindungen der Formel I.

Diese Verbindungen und Salze sind neu und zeichnen sich durch wertvolle therapeutische Eigenschaften aus. Sie eignen sich insbesondere zur Behandlung oder Verhütung von zentralnervösen Störungen, wie Depressionen, bipolare Störungen, Angstzustände, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfalle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

Gegenstand der vorliegenden Erfindung sind die Verbindungen der allgemeinen Formel I und ihre pharmazeutisch annehmbaren Säureadditionssalze per se und als pharmazeutische Wirkstoffe, Arzneimittel, enthaltend eine Verbindung der allgemeinen Formel I oder ein pharmazeutisch annehmbares Säureadditionssalz davon, die Herstellung solcher Arzneimittel, die Verwendung von Verbindungen der allgemeinen Formel I und ihrer pharmazeutisch annehmbaren Säureadditionssalze bei der Behandlung oder Verhütung von Krankheiten und Störungen der eingangs erwähnten Art bzw. zur Herstellung von Arzneimitteln zur Behandlung derartiger Krankheiten und Störungen, sowie die Herstellung der Verbindungen der obigen Formel I und ihrer pharmazeutisch annehmbaren Säureadditionssalze und hierfür geeignete Zwischenprodukte.

Der Ausdruck "nieder" bezeichnet Reste und Verbindungen mit höchstens sieben, vorzugsweise höchstens vier Kohlenstoffatomen. Der Ausdruck "Alkyl" bezeichnet geradkettige oder verzweigte, gesättigte Kohlenwasserstoffeste, wie Methyl, Ethyl, Isopropyl oder t-Butyl. Der Ausdruck "Alkoxy" bezeichnet über ein Sauerstoffatom gebundene Alkylgruppen, wie Methoxy, Ethoxy, Propoxy, Isopropoxy oder Butoxy. Der Ausdruck "Alkylthio" bezeichnet über ein Schwefelatom gebundene Alkylgruppen wie Methylthio oder Ethylthio. Der Ausdruck "Halogen" bezeichnet die vier Formen F, Cl, Br, J.

Der Ausdruck "pharmazeutisch annehmbare Säureadditionssalze" umfasst Salze mit anorganischen und organischen Säuren, wie Salzsäure, Bromwasserstoffsäure, Salpetersäure, Schwefelsäure, Phosphorsäure, Zitronensäure, Ameisensäure, Fumarsäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Bevorzugte Verbindungen der Formel I sind solche, worin $R^3$ niederes Alkoxy bedeutet, insbesondere solche, worin $R^1$ Wasserstoff oder Halogen und $R^2$ Halogen bedeuten.

Weitere bevorzugte Verbindungen sind solche, worin $R^3$ Wasserstoff und $R^1$ und $R^2$ je Halogen bedeuten.

Ganz besonders bevorzugte Verbindungen sind:

9-Chlor-8-fluor-1,2,3,4-tetrahydro-10-methoxy-pyrazino[1,2-a]indol;
8-Fluor-10-methoxy-1,2,3,4-tetrahydropyrazino[1,2-a]indol;
9-Fluor-10-methoxy-1,2,3,4-tetrahydropyrazino[1,2-a]indol;
9-Brom-1,2,3,4-tetrahydro-10-methoxy-pyrazino[1,2-a]indol; und
9-Chlor-8-fluor-1,2,3,4-tetrahydropyrazino[1,2-a]indol.

Weitere Beispiele für Verbindungen der Formel I sind:
8-Chlor-10-methoxy-1,2,3,4-tetrahydropyrazino[1,2-a]indol;
7-Chlor-1,2,3,4-tetrahydro-10-methoxy-pyrazino[1,2-a]indol; und
8-Brom-1,2,3,4-tetrahydro-10-methoxy-pyrazino[1,2-a]indol.

Die Verbindungen der Formel I und deren pharmazeutisch verwendbare Säureadditionssalze lassen sich erfindungsgemäss herstellen, indem man eine Verbindung der allgemeinen Formel

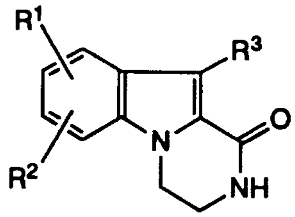

II

worin $R^1$, $R^2$ und $R^3$ obige Bedeutung haben,
reduziert und erwünschtenfalls eine erhaltene Verbindung der Formel I in ein pharmazeutisch annehmbares Säureadditionssalz überführt.

Die Reduktion wird vorzugsweise durch Behandeln mit Lithiumaluminiumhydrid oder ähnlichen Reduktionsmitteln, wie Diboran u.dgl., durchgeführt. Die Umsetzung erfolgt in einem inerten Lösungsmittel, wie z.B. Tetrahydrofuran, in einem Temperaturbereich von Raumtemperatur bis Siedetemperatur, vorzugsweise bei Siedetemperatur.

Als pharmazeutisch annehmbare Säureadditionssalze der Verbindungen der Formel I kommen sowohl Salze mit anorganischen als auch Salze mit organischen Säuren in Betracht. Beispiele derartiger Salze sind Hydrochloride, Hydrobromide, Nitrate, Sulfate, Phosphate, Citrate, Formiate, Fumarate, Maleate, Acetate, Succinate, Tartrate, Methansulfonate, p-Toluolsulfonate und dergleichen. Diese Salze können nach an sich bekannten und jedem Fachmann geläufigen Methoden hergestellt werden.

Die verschiedenen als Ausgangsstoffe verwendeten Verbindungen können beispielsweise gemäss den nachfolgenden Reaktionsschemata und den jeweils daran anschliessenden Beschreibungen der verschiedenen Reaktionen hergestellt werden.

Reaktionsschema I

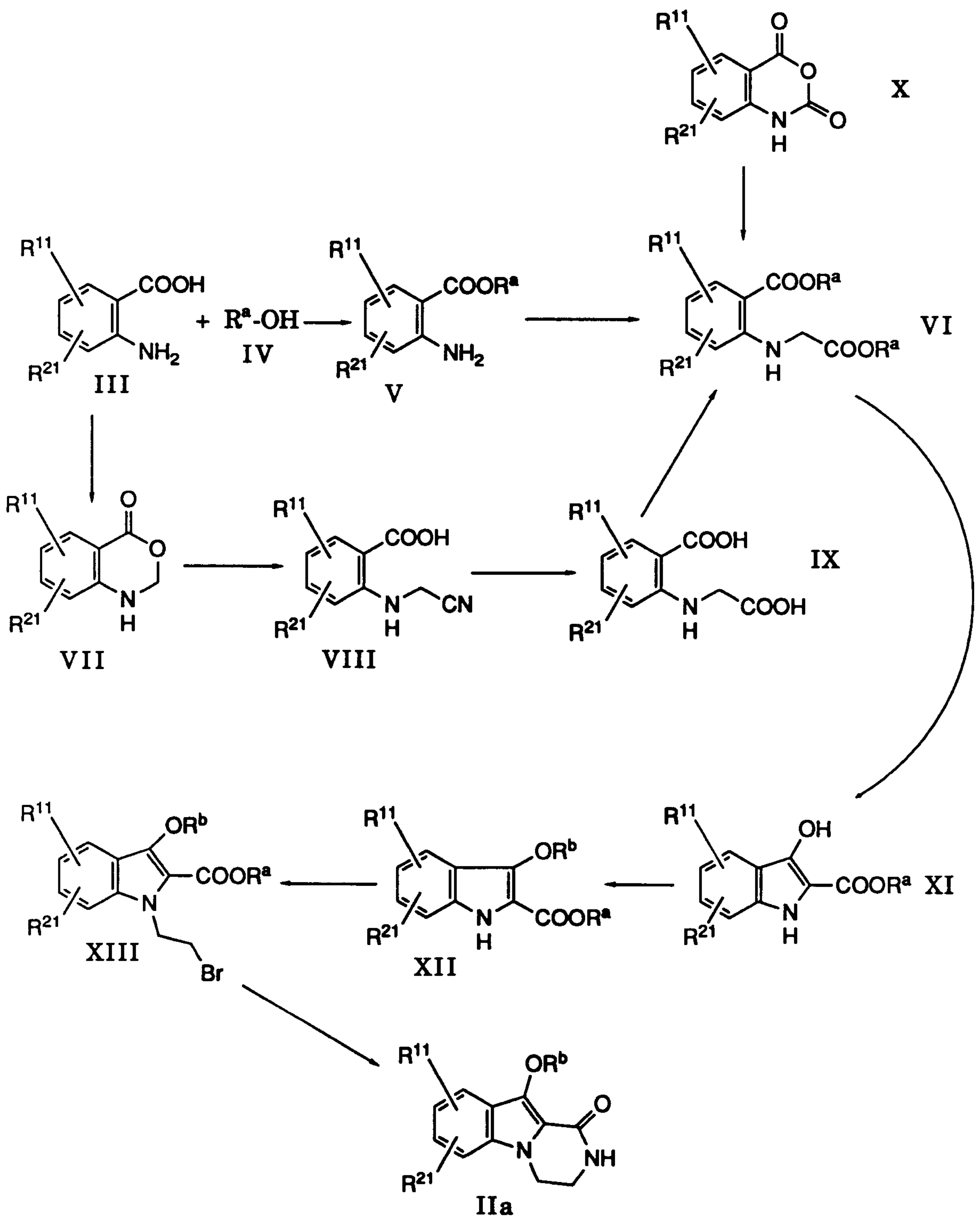


$R^{11}$ bedeutet Wasserstoff, Halogen, Trifluormethyl, niederes Alkyl, Hydroxy oder niederes Alkoxy, $R^{21}$ bedeutet Wasserstoff oder Halogen und $R^a$ und $R^b$ bedeuten je niederes Alkyl.

Durch Umsetzung einer Anthranilsäure der Formel III mit einem Alkohol der Formel IV erhält man einen entsprechenden Ester der Formel V. Die Ester der Formel V sind aus Verbindungen der Formeln III und IV nach geläufigen Methoden zugänglich, siehe beispielsweise Tetrahedron 33, 217(1977), oder können auf analoge Weise hergestellt werden.

Durch Behandeln eines Esters der Formel V mit einem α-Halogencarbonsäurealkylester, wie beispielsweise α-Bromessigsäureethylester, erhält man eine Verbindung der Formel VI. Zweckmässigerweise dient der α-Halogencarbonsäurealkylester gleichzeitig als Reagenz und als Lösungsmittel, wobei ein Carbonat, beispielsweise Kaliumcarbonat, in äquimolarer Menge beigegeben wird. Diese Reaktion wird vorzugsweise in einem Temperaturbereich von ca. 20°C bis ca. 80°C durchgeführt, analog einem in Pr.roy.Soc. 148, 481(1958) beschriebenen Verfahren.

Die Verbindungen der Formel VI können aber auch dadurch hergestellt werden, dass man zuerst eine Anthranilsäure der Formel III mit Formaldehyd in eine Verbindung der Formel VII umwandelt, wobei diese Umsetzung zweckmässigerweise in einem unter den Reaktionsbedingungen inerten Lösungsmittel,wie beispielsweise niederen Alkoholen, vorzugsweise Methanol, durchgeführt wird. Anschliessend wird die so erhaltene Verbindung der Formel VII mit einem Cyanid, wie beispielsweise Kaliumcyanid, in einem polaren Lösungsmittel, vorzugsweise Wasser, und bei einer Reaktionstemperatur von etwa 60°C in eine Verbindung der Formel VIII überführt. Durch Behandeln einer Verbindung der Formel VIII mit wässriger Lauge, beispielsweise Natronlauge, gelangt man zu Verbindungen der Formel IX. Diese Umsetzung erfolgt bevorzugt in einem Temperaturbereich von ca. 100°C bis ca. 120°C. Die Verbindungen der Formel IX können dann mit Alkoholen nach an sich bekannten und jedem Fachmann geläufigen Methoden in die Verbindungen der Formel VI umgewandelt werden.

Die Verbindungen der Formel VI können aber auch durch Umsetzung eines Isatosäureanhydrids der Formel X mit einem α-Halogenessigsäureethylester, wie beispielsweise Bromessigsäureethylester, in einem polaren Lösungsmittel, wie DMSO, und anschliessende Umsetzung mit einem Alkohol hergestellt werden.

Durch Cyclisierung einer Verbindung der Formel VI erhält man ein Indol der Formel XI. Diese Verbindungen sind bekannt, siehe beispielsweise J. Heterocyclic Chem. 16, 221(1979) oder können auf analoge Weise hergestellt werden.

Durch Umsetzung eines Indols der Formel XI mit einem Alkylierungsmittel, beispielsweise mit einem Dialkylsulfat oder mit Diazomethan, erhält man Verbindungen der Formel XII. Diese Umsetzung erfolgt in alkoholischen Lösungsmitteln, vorzugsweise Methanol, bei Raumtemperatur.

Durch Behandeln einer Verbindung der Formel XII mit 1,2-Dibromethan, erhält man eine Verbindung der Formel XIII. Diese Umsetzung wird unter den Bedingungen der Phasentransferkatalyse durchgeführt: Die Umsetzung erfolgt unter Rühren in einem zwei-Phasensystem aus Wasser und einem mit Wasser nicht mischbaren organischen Lösungsmittels in Gegenwart einer starken Base und eines Phasentransferkatalysators. Zweckmässigerweise kann als organisches Lösungsmittel 1,2-Dibromethan, welches gleichzeitig als Reagenz dient, eingesetzt werden Als starke Base eignet sich zum Beispiel Kaliumhydroxid oder Natriumhydroxid. Es können die üblichen Phasentransferkatalysatoren eingesetzt werden. Geeignete Katalysatoren sind zum Beispiel Benzyltrimethylammoniumchlorid, Tetrabutylammoniumbromid und ähnliche Verbindungen. Die Reaktion wird vorzugsweise in einem Temperaturbereich von ca. 20°C bis ca. 80°C durchgeführt.

Durch Cyclisierung einer Verbindung der Formel XIII mit Ammoniak erhält man eine Verbindung der Formel IIa, d. h. eine Verbindung der Formel II, worin $R^3$ niederes Alkoxy bedeutet. Die Reaktion wird in einem Autoklaven bei Reaktionstemperaturen von 80 - 100°C, vorzugsweise bei 80°C durchgeführt.

Reaktionsschema II

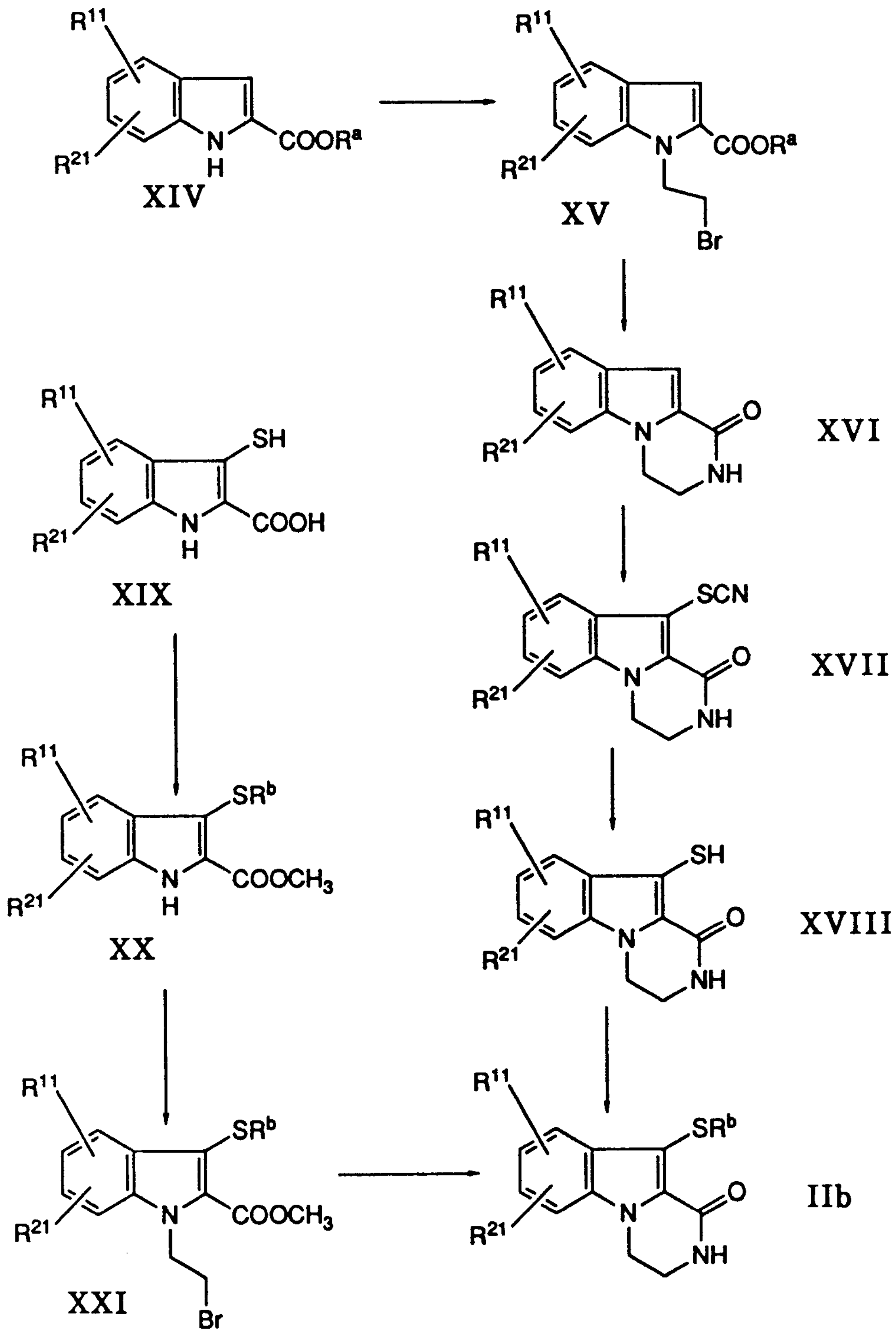

XIV
XV
XVI
XIX
XVII
XX
XVIII
XXI
IIb

$R^{11}$, $R^{21}$, $R^a$ und $R^b$ besitzen die oben angegebene Bedeutung.

Durch Behandeln eines Indols der Formel XIV mit 1,2-Dibromethan, erhält man eine Verbindung der Formel XV. Diese Umsetzung erfolgt unter den Bedingungen der Phasentransferkatalyse, wie sie oben beschrieben worden ist, nämlich im Zusammenhang mit der Herstellung der Verbindungen der Formel XIII aus den Verbindungen der Formel XII.

Die Verbindungen der Formel XIV sind bekannt, siehe beispielsweise Synthesis 1985, 186, oder können auf analoge Weise hergestellt werden.

Durch Cyclisierung einer Verbindung der Formel XV mit Ammoniak erhält man eine Verbindung der Formel XVI. Die Reaktion wird in einem Autoklaven bei Reaktionstemperaturen von 50 bis 100 ° C, vorzugsweise bei 80 ° C, durchgeführt.

Falls in Formel XVI $R^{11}$ und $R^{21}$ von Wasserstoff verschieden sind, so handelt es sich um eine Verbindung der Formel II, worin $R^3$ Wasserstoff bedeutet (wobei definitionsgemäss $R^1$ und $R^2$ beide von Wasserstoff verschieden sein müssen), und durch Reduktion kann daraus erfindungsgemäss eine entsprechende Verbindung der Formel I hergestellt werden.

Durch Umsetzung einer Verbindung der Formel XVI mit einem Alkalithiocyanat, beispielsweise Kaliumthiocyanat, und Brom in einem Alkohol, wie beispielsweise Methanol, erhält man eine Verbindung der Formel XVII. Diese Umsetzung erfolgt in einem Temperaturbereich von -70 ° C bis Raumtemperatur analog der in J. Am. Chem. Soc. 82, 2742(1960) beschriebenen Methode.

Durch Behandeln einer Verbindung der Formel XVII mit einer Base, beispielsweise Natriumhydroxid, in einem pH-Bereich von 10 bis 14 und einem Temperaturbereich von 80 ° C bis 100 ° C erhält man eine Verbindung der Formel XVIII. Diese Umsetzung erfolgt in polaren Lösungsmitteln, vorzugsweise Wasser/Alkohol-Mischungen.

Durch Umsetzung einer Verbindung der Formel XVIII mit einem Alkylierungsmittel, beispielsweise mit einem Dialkylsulfat oder mit Diazomethan, erhält man eine Verbindung der Fomel IIb, d. h. eine Verbindung der Formel II, worin $R^3$ niederes Alkylthio bedeutet.

Die Verbindungen der Formel IIb können aber auch aus 3-Mercapto-2-indolcarbonsäuren der Formel XIX hergestellt werden. Durch Umsetzung einer Verbindung der Formel XIX mit einem Alkylierungsmittel, beispielsweise mit einem Dialkylsulfat oder mit Diazomethan, erhält man eine Verbindung der Formel XX. Die Verbindungen der Formel XX sind bekannt, siehe beispielsweise J. Am. Chem. Soc. 82, 2742(1960) oder können auf analoge Weise hergestellt werden.

Durch Behandeln von Verbindungen der Formel XX mit 1,2-Dibromethan erhält man eine Verbindung der Formel XXI. Diese Umsetzung erfolgt unter den Bedingungen der Phasentransferkatalyse, wie sie oben beschrieben worden ist, nämlich im Zusammenhang mit der Herstellung der Verbindungen der Formel XIII aus den Verbindungen der Formel XII.

Durch Cyclisierung einer Verbindung der Formel XXI mit Ammoniak erhält man eine Verbindung der Formel IIb, d.h. eine Verbindung der Formel II, worin $R^3$ niederes Alkylthio bedeutet.

Die als Zwischenprodukte verwendeten Verbindungen der Formel II sind neu und ebenfalls Gegenstand der vorliegenden Erfindung. Die übrigen als Ausgangsstoffe oder Zwischenprodukte verwendeten Verbindungen gehören an sich bekannten Stoffklassen an.

Wie eingangs erwähnt besitzen die Verbindungen der Formel I und ihre pharmazeutisch anwendbaren Säureadditionssalze wertvolle pharmakodynamische Eigenschaften. Sie sind in der Lage, an Serotonin-Rezeptoren zu binden und eignen sich daher zur Behandlung oder Verhütung von Krankheiten und Störungen der eingangs erwähnten Art bzw. zur Herstellung entsprechender Arzneimittel.

Die Bindung von erfindungsgemässen Verbindungen der Formel I an die Serotonin-Rezeptoren wurde durch Standardmethoden in vitro bestimmt. Die Präparate wurden gemäss den nachfolgend angegebenen Tests geprüft:

a) Für die Bindung an den $5HT_{1A}$-Rezeptor gemäss dem 3H-8-OH-DPAT-Bindungs-Test nach der Methode von J.S. Peroutka, Biol. Psychiatry 20, 971-979(1985).

b) Für die Bindung an den $5HT_{1B}$-Rezeptor gemäss dem Bindungs-Test nach der Methode von S.J. Peroutka, Brain Research 344, 167-171 (1985) bzw. M.B. Emerit et al., Biochem. Pharmacol. 34, 883-892 (1985).

c) Für die Bindung an den $5HT_{1C}$-Rezeptor gemäss dem 3H-Mesulergin-Bindungs-Test nach der Methode von A.Pazos et al., Europ. J.Pharmacol. 106, 539-546 bzw. D.Hayer, Receptor Research 8, 59-81 (1988).

d) Für die Bindung an den $5HT_2$-Rezeptor gemäss dem 3H-Metanserin-Bindungs-Test nach der Methode von J.E.Leysen, Molecular Pharmacology 21, 301-304 (1981).

Es wurden die $IC_{50}$-Werte der zu prüfenden Substanzen bestimmt, d.h. diejenige Konzentration in nMol, durch welche 50% des am Rezeptor gebundenen Liganden verdrängt werden.

Die so ermittelte Aktivität einiger erfindungsgemässer Verbindungen sowie diejenige einiger Vergleichsverbindungen ist aus der nachfolgenden Tabelle ersichtlich:

| Substanz | Testmethode | | | |
|---|---|---|---|---|
| | a | b | c | d |
| Buspiron | 19.50 | | 3700.0 | 990.0 |
| NAN-190 | 0.56 | | 1800.0 | 581.0 |
| 5HT | 1.50 | 4.33 | 9.5 | 1730.0 |
| Metergolin | 4.80 | | 5.5 | 64.9 |
| mCPP | 227.00 | 74.20 | 53.0 | 319.0 |
| RU 24969 | 8.00 | 1.84 | 159.0 | 2500.0 |
| CP 93129 | 1620.00 | 19.70 | 2780.0 | 29200.0 |
| Ritanserin | 5750.00 | | 37.0 | 3.1 |
| Pirenperon | 2870.00 | | 37.0 | 2.9 |
| A | 687.00 | | 775.0 | 12500.0 |
| B | 2370.00 | 398.00 | 367.0 | 9390.0 |
| C | 1070.00 | 327.00 | 221.0 | 5170.0 |
| D | 657.00 | 199.00 | 195.0 | 3140.0 |
| E | 407.00 | 56.10 | 83.8 | 1020.0 |
| F | 425.00 | 370.00 | 406.0 | 9460.0 |

| | | | |
|---|---|---|---|
| G | 298.00 | 269.0 | 2810.0 |
| H | 354.00 | 90.5 | 1030.0 |
| I | 106.00 | 219.0 | 2270.0 |
| J | 512.00 | 66.0 | 374.0 |
| K | 705.00 | 1350.0 | 11200.0 |
| L | 459 | 251 | 5850 |
| M | 690 | 923 | 3030 |
| N | 964 | 57 | 902 |

A = 10-Methoxy-1,2,3,4-tetrahydropyrazino[1,2-a]-indol

B = 8-Fluor-1,2,3,4-tetrahydro-10-methoxy-pyrazino[1,2-a]-indol

C = 9-Fluor-1,2,3,4-tetrahydro-10-methoxy-pyrazino[1,2-a]indol

D = 8-Chlor-1,2,3,4-tetrahydro-10-methoxy-pyrazino[1,2-a]-indol

E = 9-Brom-1,2,3,4-tetrahydro-10-methoxy-pyrazino[1,2-a]indol

F = 7-Chlor-1,2,3,4-tetrahydro-10-methoxy-pyrazino[1,2-a]indol

G = 8-Brom-1,2,3,4-tetrahydro-10-methoxy-pyrazino[1,2-a]indol

H = 9-Chlor-8-fluor-1,2,3,4-tetrahydro-10-methoxy-pyrazino[1,2-a]indol

I = 1,2,3,4-Tetrahydro-10-methoxy-9-methyl-pyrazino[1,2-a]indol

J = 9-Trifluormethyl-1,2,3,4-tetrahydro-10-methoxy-pyrazino[1,2-a]-indol

K = 1,2,3,4-Tetrahydro-10-methylthiopyrazino[1,2-a]-indol

L = 7,9-Dichlor-1,2,3,4-tetrahydro-10-methoxy-pyrazino[1,2-a]indol

M = 6-Brom-1,2,3,4-tetrahydro-10-methoxy-pyrazino[1,2-a]indol

N = 9-Chlor-8-fluor-1,2,3,4-tetrahydropyrazino[1,2-a]indol.

Einige der Verbindungen der Formel I wurden auch in Tierversuchen geprüft.

Antagonismus von mCPP-induzierten Penis-Erektionen (Ratte)

Es hat sich gezeigt, dass Penis-Erektionen von der Stimulierung von $5HT_{1c}$-Rezeptoren abhängen, vgl. Berendsen & Broekkamp, Eur. J. Pharmacol. 135, 179-184(1987). Man verabreicht die zu prüfende Substanz an mit mCCP vorbehandelte Tiere und ermittelt die Anzahl der innerhalb von 45 Minuten

auftretenden Peniserektionen. Als $ID_{50}$ bezeichnet man diejenige Dosis der Testsubstanz, welche die Anzahl dieser Erektionen um 50% hemmt.

| Substanz | $ID_{50}$ (mg/kg, s.c.) |
|---|---|
| B | 4.2 |
| C | 17.0 |
| E | 4.3 |
| I | 2.7 |
| N | 4.2 |

Antagonismus von Quipazine-induziertem Kopfschütteln (Ratte)

"Head Shakes" (Kopfschütteln) hängen von der Stimulierung von $5HT_2$-Rezeptoren ab, vgl. Goodwin & Green, Br. J. Pharmacol. 84, 743-753 (1985). Man verabreicht die zu prüfende Substanz an mit Quipazine vorbehandelte Tiere und ermittelt die Anzahl der innerhalb von 45 Minuten auftretenden "Head Shakes". Als $ID_{50}$ bezeichnet man diejenige Dosis der Testsubstanz, welche die Anzahl dieser "Head Shakes" um 50% hemmt.

| Substanz | $ID_{50}$ (mg/kg. s.c.) |
|---|---|
| E | 3.1 |
| I | 3.0 |
| N | 5.2 |

Bei der Durchführung der oben beschriebenen Tierversuche wurden die Testsubstanz B, C, E, I und N den Tieren (Ratten) in Dosen bis zu 30 mg/kg s.c. verabreicht, ohne dass dabei irgendwelche toxischen Effekte zu beobachten gewesen wären. Daraus kann geschlossen werden, dass toxisch wirkende Dosen höher als die erwähnten Dosen von 30 mg/kg s.c. sein müssen.

Die Verbindungen der Formel I und die pharmazeutisch annehmbaren Säureadditionssalze der Verbindungen der Formel I können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, parenteral, z.B. in Form von Injektionslösungen, oder nasal erfolgen.

Zur Herstellung von pharmazeutischen Präparaten können die Verbindungen der Formel I und die pharmazeutisch annehmbaren Säureadditionssalze der Verbindungen der Formel I mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oel und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Arzneimittel, enthaltend eine Verbindung der Formel I oder ein pharmazeutisch annehmbares Säureadditionssalz davon und einen therapeutisch inerten Träger, sind ebenfalls Gegenstand der vorliegenden Erfindung, ebenso ein Verfahren zu deren Herstellung, das dadurch gekennzeichnet ist, dass man eine oder mehrere Verbindungen der Formel I und/oder pharmazeutisch annehmbare Säureadditionssalze davon und gegebenenfalls einen oder mehrere andere therapeutisch wertvolle Stoffe zusammen mit einem oder

mehreren therapeutisch inerten Trägern in eine galenische Darreichungsform bringt.

Erfindungsgemäss kann man Verbindungen der allgemeinen Formel I sowie deren pharmazeutisch annehmbare Säureadditionssalze bei der Behandlung oder Verhütung Von von zentralnervösen Störungen, wie Depressionen, bipolare Störungen, Angstzustände, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung entsprechender Arzneimittel verwenden. Die Dosierung kann innerhalb weiter Grenzen variieren und ist natürlich in jedem einzelnen Fall den individuellen Gegebenheiten anzupassen. Bei oraler Verabreichung liegt die Dosis in einem Bereich von etwa 0,01 mg pro Dosis bis etwa 500 mg pro Tag einer Verbindung der allgemeinen Formel I bzw. der entsprechenden Menge eines pharmazeutisch annehmbaren Säureadditionssalzes davon, wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

a) Eine Suspension von 1.7 g (7.7 mmol) 3-Methoxy-indol-2-carbonsäureethylester in 30 ml Dibromethan wurde mit 30 mi 28%iger NaOH und 200 mg (0.6 mmol) Tetrabutylammoniumbromid versetzt. Das Gemisch wurde 1.5 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 70 ml flüssigem Ammoniak suspendiert und 24 Stunden bei 80° im Autoklaven gerührt. Nach dem Abdampfen des Ammoniaks wurde der Rückstand in Wasser aufgenommen, verrührt und abgenutscht. Man erhielt 1.6 g (96%) rohes 10-Methoxy-1,2,3,4-tetrahydropyrazino[1,2-a]indol-1-on, das ohne weitere Reinigung in die nächste Stufe eingesetzt wurde.

b) Eine Lösung von 0.65 g (3 mmol) 10-Methoxy-1,2,3,4-tetrahydro-pyrazino[1,2-a]indol-1-on in 25 ml trockenem THF wurde mit 228 mg (6 mmol) Lithiumaluminiumhydrid versetzt und zwei Stunden unter Rückfluss gekocht. Man zerstörte den Überschuss an Hydrid vorsichtig mit Wasser und gab zu dem Gemisch 30 g Natriumsulfat. Nach Filtrieren und Einengen des Filtrats wurde der Rückstand in 20 ml Ethanol gelöst, mit 10 ml gesättigter ethanolischer HCl-Lösung versetzt und die ausgefallenen Eristalle bei 0° abgenutscht. Man erhielt 0,48 g (67%) 10-Methoxy-1,2,3,4-tetrahydropyrazino[1,2-a]indol-hydrochlorid als weisse Kristalle mit Smp. 208° (Zers.).

Beispiel 2

a) Eine Suspension von 15.2 g (83 mmol) 5-Fluoranthranilsäure-ethylester und 8.8 g (83 mmol) Natriumcarbonat in 46 ml (415 mmol) Bromessigsäuremethylester wurde 18 Stunden bei 80° gerührt. Man dampfte im Vakuum ein und der Rückstand wurde mit 184 ml Wasser, 18.4 ml Ethanol und 18.4 ml einer 25%igen Ammoniaklösung versetzt. Das Gemisch wurde 2 Stunden bei Raumtemperatur gerührt. Der entstandene Niederschlag von N-[4-Fluoro-2(ethoxycarbonyl)-phenyl]-glycin-ethylester wurde mit Hexan verrührt, abgenutscht und getrocknet. Man erhielt 12.5 g (59%) N-[2-(Ethoxycarbonyl)-4-fluoro-phenyl]-glycin-ethylester als hellgelbe Kristalle mit Smp.66-67°.

b) Eine Lösung von 4.9 g (214 mmol) Natrium in 70 ml Ethanol wurde mit einer Lösung von 27.3 g (102 mmol) N-[2-(Ethoxycarbonyl)-4-fluoro-phenyl]glycin-ethylester in 200 ml Ether versetzt. Das Gemisch wurde 2 Stunden unter Rückfluss gekocht und nach dem Abkühlen mit Wasser versetzt und mit Ether extrahiert. Die wässrige Phase wurde mit Trockeneis auf pH 8 gestellt und die ausgefallenen Kristalle wurden abgenutscht, mit wenig Wasser nachgewaschen und im Trockenschrank getrocknet. Man erhielt 15.3 g (67%) rohen 5-Fluoro-3-hydroxyindol-2-carbonsäureethylester. Eine Probe wurde aus Toluol umkristallisiert und zeigte dann einen Smp. von 152 -154°.

c) Eine Suspension von 10 g (44 mmol) 5-Fluor-3-hydroxrindol-2-carbonsäureethylester in 400 ml Methanol wurde mit 150 ml einer 60%igen etherischen Diazomethanlösung versetzt. Nach einer halben Stunde wurden erneut 50 ml dieser Lösung zugegeben und das Gemisch eine weitere halbe Stunde bei

Raumtemperatur gerührt. Das Lösungsmittel wurde abdestilliert und man erhielt 11g (quant.) rohen pulvrigen 5-Fluor-3-methoxyindol-2-carbonsäureethylester. Eine kleine Probe wurde aus Toluol/Cyclohexane umkristallisiert und zeigte einen Smp. von 96-99°.

d) Eine Lösung von 8,31 g (35 mmol) 5-Fluor-3-methoxyindol-2-carbonsäureethylester in 105 ml Dibromethan wurde mit 105 ml 28%iger NaOH und 564 mg (1.75 mmol) Tetrabutylammoniumbromid versetzt. Das Gemisch wurde 18 Stunden bei Raumtemperatur gerührt. Die Phasen wurden getrennt und die wässrige Phase wurde mit Methylenchlorid extrahiert. Die vereinigten organischen Phasen wurden mit Wasser gewaschen und über Natriumsulfat getrocknet. Das Lösungsmittel wurde abdestilliert und man erhielt 11.8 g (99%) amorphen 1-(2-Bromethyl)-5-fluor-3-methoxyindol-2-carbonsäureethylester.

MS: m/e (% Basispeak): 343, 345($M^+$,58), 328, 330(14), 264(34), 250(46), 218 (83), 41(100).

e) Eine Suspension von 11.8g (34.3 mmol) 1-(2-Bromethyl)-5-fluor-3-methoxyindol-2-carbonsäureethylester in 330 ml flüssigem Ammoniak wurde 18 Stunden bei 80° im Autoklaven gerührt. Nach dem Abdampfen des Ammoniaks wurde der Rückstand in Wasser aufgenommen, verrührt und abgenutscht. Man kristallisierte aus Ethylacetat um und erhielt 4.5 g (57%) 8-Fluoro-1,2,3,4-tetrahydro-10-methoxy-pyrazino[1,2-a]indol-1-on als weisse Kristalle mit Smp. 220-223°.

f) Eine Lösung von 1.5 g (6.4 mmol) 8-Fluor-1,2,3,4-tetrahydro-10-methoxy-pyrazino[1,2-a]indol-1-on in 30 ml trockenem THF wurde mit 500 mg (13 mmol) Lithiumaluminiumhydrid versetzt und zwei Stunden unter Rückfluss gekocht. Man zerstörte den Überschuss an Hydrid vorsichtig mit Wasser, verdünnte mit 100 ml Ether und gab zu dem Gemisch 50 g Natriumsulfat. Nach Filtieren und Einengen des Filtrats wurde der Rückstand in 50 ml Ethanol gelöst, mit 20 ml gesättigter ethanolischer HCl-Lösung versetzt und eine halbe Stunde bei Raumtemperatur gerührt, wobei Kristalle ausfielen. Man erhielt 1.2 g (73%) 8-Fluor-1,2,3,4-tetrahydro-10-methoxy-pyrazino[1,2-a]indol-hydrochlorid als weisse Kristalle mit Smp. 219-221°.

## Beispiel 3

a) Eine Suspension von 11.2 g (65 mmol) 6-Fluoranthranilsäuremethylester und 6.9 g (65 mmol) Natriumcarbonat in 36 ml (325 mmol) Bromessigsäuremethylester wurde 18 Stunden bei 80° gerührt. Man dampfte im Vakuum ein und der Rückstand wurde mit 180 ml Wasser, 18 ml Ethanol und 18 ml einer 25%igen Ammoniaklösung versetzt. Das Gemisch wurde 2 Stunden bei Raumtemperatur gerührt. Man erhielt 14.6g (91.2%) eines Niederschlages von N-[3-Fluoro-2-(methoxycarbonyl)-phenyl)-glycin-methylester, der ohne weitere Reinigung in die nächste Stufe eingesetzt wurde.

b) Eine Lösung von 2.7 g (117 mmol) Natrium in 40 ml Methanol wurde mit einer Lösung von 13.5 g (56 mmol) N-[-3-Fluor-2(methoxycarbonyl)-phenyl]-glycin-methylester in 110 ml Ether versetzt. Das Gemisch wurde 2 Stunden unter Rückfluss gekocht und nach dem Abkühlen mit Wasser versetzt und mit Ether extrahiert. Die wässrige Phase wurde mit Trockeneis auf pH 8 gestellt, die ausgefallenen Kristalle wurden abgenutscht und mit wenig Wasser nachgewaschen. Der Rückstand wurde in 400 ml Methanol suspendiert und mit 150 ml einer 60%igen etherischen Diazomethanlösung versetzt. Nach einer halben Stunde wurden erneut 50 ml dieser Lösung zugegeben und das Gemisch eine weitere halbe Stunde bei Raumtemperatur gerührt. Das Lösungsmittel wurde abdestilliert. Der Rückstand wurde mit einer Mischung aus 60ml n-Hexan und 60 ml Ether verrührt und die Kristalle wurden abgenutscht. Man erhielt 7.6 g (61%) 4-Fluor-3-methoxy-indol-2-carbonsäure methylester als weisse Kristalle mit Smp. 137-139°.

c) Eine Suspension von 7.6 g (34 mmol) 4-Fluor-3-methoxy-indol-2-carbonsäuremethylester in 170 ml Dibromethan wurde mit 85 ml 28%iger NaOH und 564 mg (1.75 mmol) Tetrabutylammoniumbromid versetzt. Das Gemisch wurde 1.5 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 340 ml flüssigem Ammoniak suspendiert und 24 Stunden bei 80° im Autoklaven gerührt. Nach dem Abdampfen des Ammoniaks wurde der Rückstand in Wasser aufgenommen, verrührt und abgenutscht. Man kristallisierte aus Alkohol um und erhielt 4.1 g (51%) 9-Fluoro-1,2,3,4-tetrahydro-10-methoxy-pyrazino[1,2-a]indol-1-on als weisse Kristalle mit Smp. 190-193°.

d) Eine Lösung von 1.5 g (6.4 mmol) 9-Fluor-1,2,3,4-tetrahydro-10-methoxy-pyrazino[1,2-a]indol-1-on in 30 ml trockenem THF wurde mit 500 mg (13 mmol) Lithiumaluminiumhydrid versetzt und zwei Stunden unter Rückfluss gekocht. Man zerstörte den Überschuss an Hydrid vorsichtig mit Wasser, verdünnte mit 100 ml Ether und gab zu dem Gemisch 50 g Natriumsulfat. Nach Filtieren und Einengen des Filtrats wurde der Rückstand in 50 ml Ethanol gelöst, mit 20 ml gesättigter ethanolischer HCl-Lösung versetzt und eine Stunde bei 0° gerührt, wobei Kristalle ausfielen. Man erhielt 1,3 g (79%) 9-Fluor-1,2,3,4-tetrahydro-10-methoxy-pyrazino[1,2-a]indol-hydrochlorid als weisse Kristalle mit Smp. 227-231°.

Beispiel 4

a) Eine Suspension von 12 g (64.6 mmol) 5-Chloranthranilsäuremethylester und 6.9 g (65 mmol) Natriumcarbonat in 36 ml (323 mmol) Bromessigsäureethylester wurde 18 Stunden bei 80° gerührt. Man dampfte im Vakuum ein und der Rückstand wurde mit 180 ml Wasser, 18 ml Ethanol und 18 ml einer 25%igen Ammoniaklösung versetzt. Das Gemisch wurde 2 Stunden bei Raumtemperatur gerührt. Der entstandene Niederschlag wurde aus Ethanol/Wasser umkristallisiert und man erhielt 11.2 g (64%) N-[4-Chloro-2-(methoxycarbonyl)-phenyl]-glycin-ethylester als weisse Nadeln mit Smp. 82-83°.
b) Eine Lösung von 1.25 g (54.4 mmol) Natrium in 20 ml Ethanol wurde mit einer Lösung von 6.87 g (25.3 mmol) N-[4-Chlor-2-(methoxycarbonyl)-phenyl]-glycinethylester in 20 ml Ether versetzt. Das Gemisch wurde 2 Stunden unter Rückfluss gekocht und nach dem Abkühlen mit Wasser versetzt und mit Ether extrahiert. Die wässrige Phase wurde mit Trockeneis auf pH 8 gestellt und die ausgefallenen Kristalle wurden abgenutscht, mit wenig Wasser nachgewaschen und im Trockenschrank getrocknet. Man erhielt 4.8 g (79%) rohen 5-Chlor-3-hydroxyindol-2-carbonsäureethylester. Eine Probe wurde aus Toluol umkristallisiert und zeigte dann einen Smp. von 172-174°.
c) Eine Suspension von 2.6 g (10.8 mmol) 5-Chlor-3-hydroxyindol-2-carbonsäureethylester in 100 ml Methanol wurde mit 100 ml einer 60%igen etherischen Diazomethanlösung versetzt. Nach einer halben Stunde wurden erneut 50 ml dieser Lösung zugegeben und das Gemisch eine weitere halbe Stunde bei Raumtemperatur gerührt. Das Lösungsmittel wurde abdestilliert und man erhielt 2.7 g (quant.) rohen pulvrigen 5-Chlor-3-methoxyindol-2-carbonsäureethylester. Eine kleine Probe wurde aus Essigester/Hexan umkristallisiert und zeigte einen Smp. von 125-127°.
d) Eine Lösung von 1.5 g (5.9 mmol) 5-Chlor-3-methoxyindol-2-carbonsäureethylester in 30 ml Dibromethan wurde mit 30 ml 28%iger NaOH und 100 mg (0.3 mmol) Tetrabutylammoniumbromid versetzt. Das Gemisch wurde 1 Stunde bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 80 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man kristallisierte aus Ethylacetat um und erhielt 0.8 g (54.4%) 8-Chlor-1,2,3,4-tetrahydro-10-methoxy-pyrazino[1,2-a]indol-1-on als weisse Kristalle mit Smp. 218-220°.
e) Eine Lösung von 0.6 g (2.4 mmol) 8-Chlor-1,2,3,4-tetrahydro-10-methoxy-pyrazino[1,2-a]indol-1-on in 25 ml trockenem THF wurde mit 200 mg (5.2 mmol) Lithiumaluminiumhydrid versetzt und zwei Stunden unter Rückfluss gekocht. Man zerstörte den Überschuss an Hydrid vorsichtig mit Wasser, verdünnte mit 100 ml Ether und gab zu dem Gemisch 30 g Natriumsulfat. Nach Filtieren und Einengen des Filtrats wurde der Rückstand in 10 ml Ethanol gelöst, mit 5 ml gesättigter ethanolischer HCl-Lösung versetzt und eine halbe Stunde bei Raumtemperatur gerührt, wobei Kristalle ausfielen. Man erhielt 0.48 g (73%) 8-Chlor-1,2,3,4-tetrahydro-10-methoxy-pyrazino[1,2-a]indol-hydrochlorid als gelbliche Kristalle mit Smp. 234-235°

Beispiel 5

a) Eine Suspension von 15.5 g (63.5 mmol) 6-Bromanthranilsäure-ethylester und 6.7 g (63.5 mmol) Natriumcarbonat in 35 ml (318 mmol) Bromessigsäureethylester wurde 18 Stunden bei 800 gerührt. Man dampfte im Vakuum ein und der Rückstand wurde mit 180 ml Wasser, 18 ml Ethanol und 18 ml einer 25%igen Ammoniaklösung versetzt. Das Gemisch wurde 2 Stunden bei Raumtemperatur gerührt. Die Emulsion wurde mit 350 ml Ether extrahiert und die organische Phase wurde dreimal mit je 175 ml Wasser und mit 35 ml gesättigter Kochsalzlösung gewaschen. Nach Trocknen und Abdestillieren erhielt man 20.3 g (96.8%) N-[3-Brom-2-(ethoxycarbonyl)-phenyl]-glycinethylester als oranges Öl, das ohne weitere Reinigung in die nächste Stufe eingesetzt wurde.
b) Eine Lösung von 1.6 g (69 mmol) Natriumin 60 ml Ethanol wurde mit einer Lösung von 19 g (57.5 mmol) N-[3-Brom-2-(ethoxycarbonyl)-phenyl]-glycin ethylester in 60 ml Ether versetzt. Das Gemisch wurde 2 Stunden bei Raumtemperatur gerührt, die gelbe Suspension wurde mit 580 ml Wasser verdünnt und mit Trockeneis auf pH 8 gestellt. Die ausgefallenen Kristalle wurden abgenutscht, mit wenig Wasser nachgewaschen und im Vakuum bei 30° getrocknet. Man erhielt 14.7 g (90%) 4-Brom-3-hydroxyindol-2-carbonsäureethylester als beige Kristalle mit Smp. 155-160°.
c) Eine Suspension von 8.5 g (30 mmol) 4-Brom-3-hydroxyindol-2-carbonsäureethylester in 300 ml Methanol wurde mit 150 ml einer 60%igen etherischen Diazomethanlösung versetzt. Nach einer halben Stunde wurden erneut 50 ml dieser Lösung zugegeben und das Gemisch eine weitere halbe Stunde bei

Raumtemperatur gerührt. Das Lösungsmittel wurde abdestilliert und der Rückstand wurde in 60 ml n-Hexan verrührt. Man erhielt 7 g (79%) 4-Brom-3-methoxyindol-2-carbonsäureethylester als weisse Kristalle mit Smp. von 137-139°.

d) Eine Lösung von 7 g (23.5 mmol) 4-Brom-3-methoxyindol-2-carbonsäureethylester in 117 ml Dibromethan wurde mit 59 ml 28%iger NaOH und 380 mg (1.17 mmol) Tetrabutylammoniumbromid versetzt. Das Gemisch wurde 6 Stunden bei Raumtemperatur gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert, der Rückstand in 230 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man erhielt 6.37 g (92%) rohes 9-Brom-1,2,3,4-tetrahydro-10-methoxy-pyrazino[1,2-a]indol-1-on. Eine Probe wurde aus Ethanol umkristallisiert und zeigte dann einen Smp. von 205-206°.

e) Eine Lösung von 4.04 g (13.7 mmol) 9-Brom-1,2,3,4-tetrahydro-10-methoxy-pyrazino[1,2-a]indol-1-on in 130 ml trockenem THF wurde mit 1 g (27 mmol) Lithiumaluminiumhydrid versetzt und zwei Stunden unter Rückfluss gekocht. Man zerstörte den Überschuss an Hydrid vorsichtig mit Wasser und gab zu dem Gemisch 13 g Natriumsulfat. Nach Filtrieren und Einengen des Filtrats wurde der Rückstand in 55 ml Ethanol gelöst, mit 30 ml gesättigter ethanolischer HCl-Lösung versetzt. Die Kristalle wurden abgenutscht und aus Methanol umkristallisiert. Man erhielt 1.56 g (36%) 9-Brom-1,2,3,4-tetrahydro-10-methoxy-pyrazino[1,2-a]indol-hydrochlorid als weisse Kristalle mit Smp. 236-238° (Zers.)

Beispiel 6

a) Eine Suspension von 10 g (50 mmol) 4-Chloranthranilsäure-ethylester und 5.3 g (50 mmol) Natriumcarbonat in 20 ml (180 mmol) Bromessigsäureethylester wurde 18 Stunden bei 80° gerührt. Nach dem Abkühlen versetzte man mit 20 ml Ethanol und 20 ml einer 25%igen Ammoniaklösung. Man erhielt 10 g (70%) eines Niederschlages von N-[4-Chlor-2-(ethoxycarbonyl)-phenyl]-glycin-ethylester, der ohne weitere Reinigung in die nächste Stufe eingesetzt wurde.

b) Eine Lösung von 2.03 g (88.2 mmol) Natrium in 30 ml Ethanol wurde mit einer Lösung von 12 g (42 mmol) N-[5-Chlor-2-(ethoxycarbonyl)-phenyl]-glycin-ethylester in 150 ml Ether versetzt. Das Gemisch wurde 2 Stunden unter Rückfluss gekocht und nach dem Abkühlen mit Wasser versetzt und mit Ether extrahiert. Die wässrige Phase wurde mit Trockeneis auf pH 8 gestellt und die ausgefallenen Kristalle wurden abgenutscht. Es wurde mit wenig Wasser nachgewaschen und im Vakuum bei 50° getrocknet. Man erhielt 6.15 g (61%) 6-Chlor-3-hydroxyindol-2-carbonsäureethylester mit Smp. 167-170°.

c) Eine Suspension von 3 g (12.5 mmol) 6-Chlor-3-hydroxyindol-2-carbonsäureethylester in 125 ml Methanol wurde mit 100 ml einer 60%igen etherischen Diazomethanlösung versetzt. Nach einer halben Stunde wurden erneut 50 ml dieser Lösung zugegeben und das Gemisch eine weitere halbe Stunde bei Raumtemperatur gerührt. Das Lösungsmittel wurde abdestilliert und man erhielt 3.27 g (94.5%) 6-Chlor-3-methoxyindol-2-carbonsäureethylester, der ohne weitere Reinigung in die nächste Stufe eingesetzt wurde.

d) Eine Lösung von 3.27 g (12.5 mmol) 6-Chlor-3-methoxyindol-2-carbonsäureethylester in 60 ml Dibromethan wurde mit 60 ml 28%iger NaOH und 200 mg (0.6 mmol) Tetrabutylammoniumbromid versetzt. Das Gemisch wurde 2 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 80 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man kristallisiert aus Ethanol um und erhielt 2.4 g (74.3%) 7-Chlor-1,2,3,4-tetrahydro-10-methoxy-pyrazino[1,2-a]indol-1-on als weisse Kristalle mit Smp. 218-220°.

e) Eine Lösung von 0.95 g (3.7 mmol) 7-Chlor-1,2,3,4-tetrahydro-10-methoxy-pyrazino[1,2-a]indol-1-on in 40 ml trockenem THF wurde mit 0.28 g (7.5 mmol) Lithiumaluminiumhydrid versetzt und zwei Stunden unter Rückfluss gekocht. Man zerstörte den Überschuss an Hydrid vorsichtig mit Wasser und gab zu dem Gemisch 30 g Natriumsulfat. Nach Filtrieren und Einengen des Filtrats wurde der Rückstand in 20 ml Ethanol gelöst und mit 10 ml gesättigter ethanolischer HCl-Lösung versetzt. Die Kristalle wurden abgenutscht und aus Ethanol umkristallisiert. Man erhielt 0.35 g (34%) 7-Chlor-1,2,3,4-tetrahydro-10-methoxy-pyrazino[1,2-a]indol-hydrochlorid als weisse Kristalle mit Smp. 228-230°.

Beispiel 7

a) Eine Suspension von 13 g (53 mmol) 5-Bromanthranilsäure-ethylester und 5.7 g (53 mmol) Natriumcarbonat in 30 ml (270 mmol) Bromessigsäureethylester wurde 40 Stunden bei 80° gerührt. Man dampfte im Vakuum ein und der Rückstand wurde mit 145 ml Wasser, 14.5 ml Ethanol und 14.5 ml einer 25%igen Ammoniaklösung versetzt. Das Gemisch wurde 2 Stunden bei Raumtemperatur gerührt. Die ausgefallenen Kristalle wurden mit Hexan gewaschen und im Vakuum bei 45° getrocknet. Man erhielt 13.08 g (74.4%) N-[4-Brom-2-(ethoxycarbonyl)-phenyl]-glycin-ethylester mit einem Smp. von 95-96°.
b) Eine Lösung von 1 g (43.5 mmol) Natrium in 50 ml Ethanol wurde mit einer Suspension von 13 g (39,3 mmol) N-[4-Brom-2-(ethoxycarbonyl)-phenyl]-glycin-ethylester in 25ml Ether und in 25 ml Ethanol versetzt. Das Gemisch wurde 2 Stunden unter Rückfluss gekocht und nach dem Abkühlen mit Wasser versetzt. Mit Trockeneis wurde auf pH 8 gestellt und die ausgefallenen Kristalle wurden abgenutscht. Man erhielt 8.3 g (74%) rohen 5-Brom-3-hydroxyindol-2-carbonsäureethylester, der ohne weitere Reinigung in die nächste Stufe eingesetzt wurde.
c) Eine Suspension von 8.2 g (29 mmol) 5-Brom-3-hydroxyindol-2-carbonsäureethylester in 300 ml Methanol wurde mit 150 ml einer 60%igen etherischen Diazomethanlösung versetzt. Nach einer halben Stunde wurden erneut 100 ml dieser Lösung zugegeben und das Gemisch eine weitere halbe Stunde bei Raumtemperatur gerührt. Das Lösungsmittel wurde abdestilliert und man erhielt 8.4 g (quant.) rohen pulvrigen 5-Brom-3-methoxyindol-2-carbonsäureethylester. Eine kleine Probe wurde aus Methanol umkristallisiert und zeigte einen Smp. von 136-138°.
d) Eine Lösung von 8.4 g (28.2 mmol) 5-Brom-3-methoxyindol-2-carbonsäureethylester in 130 ml Dibromethan wurde mit 130 ml 28%iger NaOH und 440 mg (1.3 mmol) Tetrabutylammoniumbromid versetzt. Das Gemisch wurde 4 Stunde bei Raumtemperatur gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 280 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man erhielt 6.3 g (75.7%) 8-Brom-1,2,3,4-tetrahydro-10-methoxy-pyrazino[1,2-a]indol-1-on als beige Kristalle mit Smp. 209-211°.
e) Eine Lösung von 0.9 g (3 mmol) 8-Brom-1,2,3,4-tetrahydro-10-methoxy-pyrazino[1,2-a]indol-1-on in 60 ml trockenem THF wurde mit 20 ml einer 1N Diboranlösung in THF versetzt und zwei Stunden unter Rückfluss gekocht. Man zerstörte den Überschuss an Hydrid mit 5 ml einer gesättigten ethanolischen HCl und erhitzte das Gemisch eine Stunde unter Rückfluss. Man stellte mit konzentrierter Natronlauge basisch und extrahierte mit Essigester und Wasser. Die organische Phase wurde mit Natriumsulfat getrocknet und das Lösungsmittel wurde entfernt. Der Rückstand wurde in 10 ml Ethanol gelöst und abfiltriert. Durch Zugabe von 10 ml gesättigter ethanolischer HCl-Lösung erhielt man 0.5 g (51.6%) 8-Brom-1,2,3,4-tetrahydro-10-methoxy-pyrazino[1,2-a]indol-hydrochlorid als hellgelbe Kristalle mit Smp. 239°

Beispiel 8

a) Eine Suspension von 3 g (14 mmol) 6-Chlor-5-fluoranthranilsäureethylester und 1.5 g (14 mmol) Natriumcarbonat in 8 ml (71 mmol) Bromessigsäureethylester wurde 30 Stunden bei 80° gerührt. Man dampfte im Vakuum ein und der Rückstand wurde mit 33 ml Wasser, 3.3 ml Ethanol und 3.3 ml einer 25%igen Ammoniaklösung versetzt. Das Gemisch wurde mit Essigester extrahiert und die organische Phase mit Natriumsulfat getrocknet.Das Lösungsmittel wurde abdestilliert, und man erhielt 4 g (95%) N-[3-Chlor-2-(ethoxycarbonyl)-4-flour-phenyl]-glycin-ethylester als oranges Öl, das ohne weitere Reinigung in die nächste Stufe eingesetzt wurde.
b) Eine Lösung von 0.33 g (14.3 mmol) Natrium in 15 ml Ethanol wurde mit einer Suspension von 3.6 g (11.8 mmol) N-[3-Chlor-2-(ethoxycarbonyl)-4-flour-phenyl]-glycin-ethylester in 15ml Ether versetzt. Das Gemisch wurde 1 Stunde unter Rückfluss gekocht und nach dem Abkühlen mit Wasser versetzt. Mit Trockeneis wurde auf pH 8 gestellt und die ausgefallenen Kristalle wurden abgenutscht. Man erhielt 2.7 g (88.5%) rohen 4-Chlor-5-fluor-3-hydroxyindol-2-carbonsäureetbylester. Eine Probe wurde aus Methanol umkristallisiert und zeigte dann einen Smp. von 191-192°.
c) Eine Suspension von 2 g (7.7 mmol) 4-Chlor-5-fluor-3-hydroxyindol-2-carbonsäureethylester in 70 ml Methanol wurde mit 50 ml einer 60%igen etherischen Diazomethanlösung versetzt. Nach einer halben Stunde wurden erneut 50 ml dieser Lösung zugegeben und das Gemisch eine weitere halbe Stunde bei Raumtemperatur gerührt. Das Lösungsmittel wurde abdestilliert. Der Rückstand wurde aus Methanol

umkristallisiert. Man erhielt 1.25 g (59.3%) 4-Chlor-5-fluor-3-methoxyindol-2-carbonsäureethylester als feine weisse Kristalle mit Smp. 191-192°.

d) Eine Lösung von 1.2 g (4.4 mmol) 4-Chlor-5-fluor-3-methoxyindol-2-carbonsäureethylester in 22 ml Dibromethan wurde mit 22 ml 28%iger NaOH und 44 mg (0.14 mmol) Tetrabutylammoniumbromid versetzt. Das Gemisch wurde 1.5 Stunden bei Raumtemperatur gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 45 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man erhielt 1.1 g (97.3%) 9-Chlor-8-fluor-1,2,3,4-tetrahydro-10-methoxy-pyrazino[1,2-a]indol-1-on als beige Kristalle mit Smp. 189-193°.

e) Eine Lösung von 1.1 g (4.1 mmol) 9-Chlor-8-fluor-1,2,3,4-tetrahydro-10-methoxy-pyrazino[1,2a]indol-1-on in 45 ml trockenem THF wurde mit 0.32 g (8.4 mmol) Lithiumaluminiumhydrid versetzt und zwei Stunden unter Rückfluss gekocht. Man zerstörte den Überschuss an Hydrid vorsichtig mit Wasser und gab zu dem Gemisch 5 g Natriumsulfat. Nach Filtieren und Einengen des Filtrats wurde der Rückstand in 50 ml Ethanol gelöst und mit 20 ml gesättigter ethanolischer HCl-Lösung versetzt. Man erhielt 0.55 g (63%) 9-Chlor-8-fluor-1,2,3,4-tetrahydro-10-methoxy-pyrazino[1,2-a]indol-hydrochlorid als weisse Kristalle mit Smp. 243-245°

Beispiel 9

a) Eine Lösung von 1.7 g (10 mmol) 4-Methyl-isatinsäureanhydrid in 20 ml Dimethylsulfoxid wurde mit 1 g gepulvertem KOH und mit 1.83 g (11 mmol) Bromessigsäureethylester versetzt und 3 Stunden bei Raumtemperatur gerührt. Dann wurde zu dem Gemisch 30 ml Ethanol zugesetzt und eine halbe Stunde gerührt. Man extrahierte mit Ether und Wasser. Die organische Phase wurde mit Natriumsulfat getrocknet und das Lösungsmittel wurde abdestilliert. Der Rückstand wurde im Kugelrohr bei einer Badtemperatur von 180° und einem Druck von 0.5 mm destilliert. Man erhielt 1.6 g (60%) N-[2-(Ethoxycarbonyl)-3-methyl-phenyl]-glycin-ethylester als farbloses Öl.

b) Eine Lösung von 0.6 g (26 mmol) Natrium in 35 ml Ethanol wurde mit einer Suspension von 5.3 g (20 mmol) N-[2-(Ethoxycarbonyl)-3-methyl-phenyl]-glycin-ethylester in 35 ml Ether versetzt. Das Gemisch wurde 1 Stunde unter Rückfluss gekocht und nach dem Abkühlen mit Wasser versetzt. Mit Trockeneis wurde auf pH 8 gestellt und die ausgefallenen Kristalle wurden abgenutscht. Man erhielt 3.4 g (77.6%) rohen 3-Hydroxy-4-methyl-indol-2-carbonsäureethylester. Eine Probe wurde aus Ethanol umkristallisiert und zeigte dann einen Smp. von 124-125°.

c) Eine Suspension von 2 g (9.1 mmol) 3-Hydroxy-4-methyl-indol-2-carbonsäureethylester in 20 ml Methanol wurde mit 100 ml einer 60%igen etherischen Diazomethanlösung versetzt. Nach einer halben Stunde wurden erneut 50 ml dieser Lösung zugegeben und das Gemisch eine weitere halbe Stunde bei Raumtemperatur gerührt. Das Lösungsmittel wurde abdestilliert. Der Rückstand wurde aus Ethanol umkristallisiert. Man erhielt 1.27 g (60%) 3-Methoxy-4-methyl-indol-2-carbonsäureethylester als weisse Kristalle mit Smp. 109-110°.

d) Eine Lösung von 1 g (4.3 mmol) 3-Methoxy-4-methyl-indol-2-carbonsäureethylester in 20 ml Dibromethan wurde mit 20 ml 28%iger NaOH und 44 mg (0.14 mmol) Tetrabutylammoniumbromid versetzt. Das Gemisch wurde 2 Stunden bei 80° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 45 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man erhielt 0.9 g (91%) 1,2,3,4-tetrahydro-10-methoxy-9-methyl-pyrazino[1,2-a]indol-1-on als beige Kristalle mit Smp. 201-202°.

e) Eine Lösung von 0.83 g (3.6 mmol) 1,2,3,4-tetrahydro-10-methoxy-9-methyl-pyrazino[1,2-a]indol-1-on in 45 ml trockenem THF wurde mit 0.28 g (7.5 mmol) Lithiumaluminiumhydrid versetzt und zwei Stunden unter Rückfluss gekocht. Man zerstörte den Überschuss an Hydrid vorsichtig mit Wasser und gab zu dem Gemisch 5 g Natriumsulfat. Nach Filtieren und Einengen des Filtrats wurde der Rückstand in 40 ml Ethanol gelöst und mit 20 ml gesättigter ethanolischer HCl-Lösung versetzt. Man erhielt 0.51 g (56 %) 1,2,3,4-tetrahydro-10-methoxy-9-methyl-pyrazino[1,2-a]indol hydrochlorid als weisse Kristalle mit Smp. 231-233°

Beispiel 10

a) Eine Suspension von 4.8 g (22 mmol) 6-Trifluormethylanthranilsäure methylester und 2.5 g (23.3 mmol) Natriumcarbonat in 20 ml (177.5 mmol) Bromessigsäureethylester wurde 20 Stunden bei 80° gerührt. Man dampfte im Vakuum ein und der Rückstand wurde mit 50 ml Wasser, 5 ml Ethanol und 5 ml einer 25%igen Ammoniaklösung versetzt. Das Gemisch wurde mit Dichlormethan extrahiert und die organische Phase mit Natriumsulfat getrocknet. Das Lösungsmittel wurde abdestilliert, und man erhielt 5.9 g (93%) N-[3-Triflourmethyl-2-(methoxycarbenyl)-phenyl]-glycin-methylester als oranges Öl, das ohne weitere Reinigung in die nächste Stufe eingesetzt wurde.
b) Eine Lösung von 0.43 g (18.6 mmol) Natrium in 20 ml Methanol wurde mit einer Suspension von 4.5 g (15.5 mmol) N-[3-Trifluormethyl-2-(ethoxy-carbonyl)-phenyl]-glycin-methylester in 100 ml Ether versetzt. Das Gemisch wurde 2 Stunden bei Raumtemperatur gerührt und mit Wasser versetzt. Die wässrige Phase wurde mit Trockeneis auf pH 8 gestellt und die ausgefallenen Kristalle wurden abgenutscht. Man erhielt 1.8 g (45%) 4-Trifluormethyl-3-hydroxyindol-2-carbonsäuremethylester.
c) Eine Lösung von 1.8 g (6.9 mmol) 4-Trifluormethyl-3-hydroxyindol-2-carbonsäuremethylester in 70 ml Methanol wurde mit 100 ml einer 60%igen etherischen Diazomethanlösung versetzt. Nach einer halben Stunde wurden erneut 50 ml dieser Lösung zugegeben und das Gemisch eine weitere halbe Stunde bei Raumtemperatur gerührt. Das Lösungsmittel wurde abdestilliert. Man erhielt 1.9 g (quant.) 4-Trifluormethyl-3-methoxyindol-2-carbonsäuremethylester, der ohne weitere Reinigung in die nächste Stufe eingesetzt wurde.
d) Eine Lösung von 1.9 g (6.9 mmol) 4-Trifluormethyl-3-methoxyindol-2-carbonsäuremethylester in 35 ml Dibromethan wurde mit 35 ml 28%iger NaOH und 100 mg (0.1 mmol) Tetrabutylammoniumbromid versetzt. Das Gemisch wurde 2 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 50 ml flüssigem Ammoniak suspendiert und 18 Stunden bei 80° im Autoklaven gerührt. Nach dem Abdampfen des Ammoniaks wurde der Rückstand in Wasser aufgenommen, verrührt und abgenutscht. Man erhielt 0.98 g (49.5%) 9-Trifluormethyl-1,2,3,4-tetrahydro-10-methoxy-pyrazino[1,2-a]indol-1-on.
e) Eine Lösung von 0.68 g (2.4 mmol) 9-Trifluormethyl-1,2,3,4-tetrahydro-10-methoxy-pyrazino[1,2-a]-indol-1-on in 50 ml trockenem THF wurde mit 0.36g (9.6 mmol) Lithiumaluminiumhydrid versetzt und zwei Stunden unter Rückfluss gekocht. Man zerstörte den Überschuss an Hydrid vorsichtig mit Wasser und gab zu dem Gemisch 20 g Natriumsulfat. Nach Filtieren und Einengen des Filtrats wurde der Rückstand in 40 ml Ethanol gelöst und mit 20 ml gesättigter ethanolischer HCl-Lösung versetzt. Man erhielt 0.6 g (56%) 9-Trifluormethyl-1,2,3,4-tetrahydro-10-methoxy-pyrazino[1,2-a]indol-hydrochlorid als weisse Kristalle mit Smp. 244-245°.

Beispiel 11

a) Eine Suspension von 1.7 g (7.7 mmol) 3-Methylthio-indol-2-carbonsäuremethylester in 50 ml Dibromethan wurde mit 50 ml 28%iger NaOH und 100 mg (0.3 mmol) Tetrabutylammoniumbromid versetzt. Das Gemisch wurde 2 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde in 50 ml flüssigem Ammoniak suspendiert und 24 Stunden bei 80° im Autoklaven gerührt. Nach dem Abdampfen des Ammoniaks wurde der Rückstand mit Wasser und Essigester extrahiert. Die organische Phase wurde getrocknet und eingedampft. Der Rückstand wurde aus Essigester umkristallisiert Man erhielt 0.73 g (40%) 1,2,3,4-Tetrahydro-10-methylthio-pyrazino[1,2-a]indol-1-on als weisse Kristalle mit Smp. 148-156°.
b) Eine Lösung von 0.68 g (3 mmol) 1,2,3,4-Tetrahydro-10-methylthio-pyrazino[1,2-a]indol-1-on in 30 ml trockenem THF wurde mit 228 mg (6 mmol) Lithiumaluminiumhydrid versetzt und zwei Stunden unter Rückfluss gekocht. Man zerstörte den Überschuss an Hydrid vorsichtig mit Wasser und gab zu dem Gemisch 20 g Natriumsulfat. Nach Filtieren und Einengen des Filtrats wurde der Rückstand in 10 ml Ethanol gelöst und mit 10 ml gesättigter ethanolischer HCl-Lösung versetzt. Man erhielt 0,52 g (70%) 1,2,3,4-Tetrahydro-10-methylthio-pyrazino[1,2-a]indol hydrochlorid als weisse Kristalle mit Smp. 236-237° (Zers.).

Beispiel 12

a) Eine Suspension von 5 g (22.7 mmol) 4,6-Dichloranthranilsäuremethylester und 2.4 g (22.6 mmol) Natriumcarbonat in 20 ml (179 mmol) Bromessigsäureethylester wurde 40 Stunden bei 80° gerührt. Man dampfte im Vakuum ein und der Rückstand wurde mit 90 ml Wasser, 9 ml Ethanol und 9 ml einer 25%igen Ammoniaklösung versetzt. Das Gemisch wurde eine halbe Stunde gerührt und mit 200 ml Ether extrahiert. Die organische Phase wurde mit Wasser gewaschen. Nach dem Trocknen mit Natriumsulfat und Entfernen des Lösungsmittels erhielt man 7 g (96.3%) N-[3,5-Dichlor-2-(ethoxycarbonyl)-phenyl]-glycin-ethylester als oranges Öl, das ohne weitere Reinigung in die nächste Stufe eingesetzt wird.

b) Eine Lösung von 0.6 g (26 mmol) Natrium in 20 ml Ethanol wurde mit einer Suspension von 5. g (15.6 mmol) N-[3,5-Dichlor-2-(ethoxycarbonyl)-phenyl]-glycin-ethylester in 10 ml Ether versetzt. Das Gemisch wurde 1 Stunde unter Rückfluss gekocht und nach dem Abkühlen mit Wasser versetzt. Die wässrige Phase wurde mit Trockeneis auf pH 8 gestellt und die ausgefallenen Kristalle wurden abgenutscht. Man kristallisierte aus Ethanol um und erhielt 2.2 g (51%) 3-Hydroxy-4,6-dichlor-indol-2-carbonsäureethylester als bräunliche Kristalle mit Smp. 183-185°.

c) Eine Suspension von 2 g (7.3 mmol) 3-Hydroxy-4,6-dichlor-indol-2-carbonsäureethylester in 30 ml Methanol wurde mit 100 ml einer 60%igen etherischen Diazomethanlösung versetzt. Das Gemisch wurde eine halbe Stunde bei Raumtemperatur gerührt. Das Lösungsmittel wurde abdestilliert. Der Rückstand wurde aus Methanol umkristallisiert. Man erhielt 1.5 g (71%).3-Methoxy-4,6-dichlor-indol-2-carbonsäureethylester als beige Kristalle mit einem Smp. von 173-175°.

d) Eine Lösung von 1.5 g (5.2 mmol) 3-Methoxy-4,6-dichlor-indol-2-carbonsäureethylester in 30 ml Dibromethan wurde mit 30 ml 28%iger NaOH und 100 mg (0.1 mmol) Tetrabutylammoniumbromid versetzt. Das Gemisch wurde 2 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 70 ml flüssigem Ammoniak suspendiert und 23 Stunden bei 80° im Autoklaven gerührt. Nach dem Abdampfen des Ammoniaks wurde der Rückstand in Wasser aufgenommen, verrührt und abgenutscht. Man erhielt 0.97 g (65.5%) 7,9-Dichlor-1,2,3,4-tetrahydro-10-methoxy-pyrazino[1,2-a]indol-1-on als beige Kristalle mit einem Smp. von 228-232°.

e) Eine Lösung von 0.94 g (3.3 mmol) 7,9-Dichlor-1,2,3,4-tetrahydro-10-methoxy-pyrazino[1,2-a]indol-1-on in 25 ml trockenem THF wurde mit 300 mg (7.9 mmol) Lithiumaluminiumhydrid versetzt und 2 Stunden unter Rückfluss gekocht. Man zerstörte den Überschuss an Hydrid vorsichtig mit Wasser, verdünnte mit 20 ml THF und gab zu dem Gemisch 9 g Natriumsulfat. Es wurde filtriert und eingeengt. Der Rückstand wurde in 20 ml Ethanol gelöst. Durch Zugabe von 10 ml gesättigter ethanolischer HCl-Lösung wurde das Salz gefällt. Man erhielt 0.65 g (73 %) 7,9-Dichlor-1,2,3,4-tetrahydro-10-methoxy-pyrazino[1,2-a]indol hydrochlorid als weisse Kristalle, die sich über 245° zersetzen.

Beispiel 13

a) Eine Lösung von 2.4 g (8.6 mmol) N-[6-Brom-2-(carboxy)-phenyl]-glycin in 100 ml Methanol wurde mit 150 ml einer 60%igen etherischen Diazomethanlösung versetzt. Nach 30 min wurden weitere 50 ml dieser Diazomethanlösung zugegeben und die Reaktionslösung wurde 15 min gerührt. Die Lösung wurde eingedampft und man erhielt 2.6 g (quant.) N-[6-Brom-2-(methoxycarbonyl)-phenyl]-glycin-methylester als hellbraunes Öl, das ohne weitere Reinigung in die nächste Stufe eingesetzt wurde.

b) Eine Lösung von 0.25 g (10.6 mmol) Natrium in 12 ml Methanol wurde mit einer Suspension von 2.6. g (8.8 mmol) N-[6-Brom-2-(methoxycarbonyl)-phenyl]-glycin-methylester in 12 ml Ether versetzt. Das Gemisch wurde 1 Stunde unter Rückfluss gekocht und nach dem Abkühlen mit Wasser versetzt. Die wässrige Phase wurde mit Trockeneis auf pH 8 gestellt und die ausgefallenen Kristalle wurden abgenutscht. Man erhielt 2 g (86 %) 7-Brom-3-Hydroxy-indol-2-carbonsäuremethylester als beige Kristalle mit Smp. 255-258°.

c) Eine Suspension von 1.9 g (7. mmol) 7-Brom-3-Hydroxy-indol-2-carbonsäuremethylester in 120 ml Methanol wurde mit 150 ml einer 60%igen etherischen Diazomethanlösung versetzt. Das Gemisch wurde eine halbe Stunde bei Raumtemperatur gerührt. Das Lösungsmittel wurde abdestilliert. Man erhielt 2 g (quant.).7-Brom-3-Methoxy-indol-2-carbonsäuremethylester als braunes Öl, das ohne weitere Reinigung in die nächste Stufe eingesetzt wird.

d) Eine Lösung von 3.73 g (13.1 mmol) 7-Brom-3-Methoxy-indol-2-carbonsäuremethylester in 65 ml Dibromethan wurde mit 65 ml 28%iger NaOH und 100 mg (0.1 mmol) Tetrabutylammoniumbromid

versetzt. Das Gemisch wurde 2 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der feste Rückstand wurde in 70 ml flüssigem Ammoniak suspendiert und 23 Stunden bei 80° im Autoklaven gerührt. Nach dem Abdampfen des Ammoniaks wurde der Rückstand in Wasser aufgenommen, verrührt und abgenutscht. Man erhielt 0.97 g (65.5%) 6-Brom-1,2,3,4-tetrahydro-10-methoxy-pyrazino[1,2-a]indol-1-on als beige Kristalle mit einem Smp. von 148-150°.

e) Eine Lösung von 0.9 g (3 mmol) 6-Brom-1,2,3,4-tetrahydro-10-methoxy-pyrazino[1,2-a]indol-1-on in 60 ml trockenem THF wurde mit 60 ml einer 1N Diboranlösung in THF versetzt und zwei Stunden unter Rückfluss gekocht. Man zerstörte den Überschuss an Hydrid mit 5 ml einer gesättigten ethanolischen HCl und erhitzte das Gemisch eine Stunde unter Rückfluss. Man stellte mit konzentrierter Natronlauge basisch und extrahierte mit Essigester und Wasser. Die organische Phase wurde mit Natriumsulfat getrocknet und das Lösungsmittel wurde entfernt. Der Rückstand wurde in 30 ml Ethanol gelöst und durch Zugabe von 10 ml gesättigter ethanolischer HCl-Lösung bei 0° erhielt man 0.5 g (51.6%) 6-Brom-1,2,3,4-tetrahydro-10-methoxy-pyrazino[1,2-a]indol-hydrochlorid als weisse Kristalle mit Smp. 226°

Beispiel 14

a) Eine Suspension von 2 g (9.3 mmol) 4-Chlor-5-fluoranthranilsäureethylester und 1 g (9.3 mmol) Natriumcarbonat in 8 ml (71 mmol) Bromessigsäureethylester wurde 30 Stunden bei 80° gerührt. Man dampfte im Vakuum ein und der Rückstand wurde mit 33 ml Wasser, 3.3 ml Ethanol und 3.3 ml einer 25%igen Ammoniaklösung versetzt. Das Gemisch wurde mit Essigester extrahiert und die organische Phase mit Natriumsulfat getrocknet. Das Lösungsmittel wurde abdestilliert, und man erhielt 2.75 g (98.5 %) N-[5-Chlor-2-(ethoxycarbenyl)-4-fluor-phenyl]-glycin-ethylester als oranges Öl, das ohne weitere Reinigung in die nächste Stufe eingesetzt wurde.

b) Eine Lösung von 0.25 g (10.9 mmol) Natrium in 11.5 ml Ethanol wurde mit einer Suspension von 2.75 g (9 mmol) N-[5-Chlor-2-(ethoxycarbonyl)-4-flour-phenyl]-glycin-ethylester in 15ml Ether versetzt. Das Gemisch wurde 2 Stunden unter Rückfluss gekocht und nach dem Abkühlen mit Wasser versetzt. Mit Trockeneis wurde auf pH 8 gestellt und die ausgefallenen Kristalle wurden abgenutscht. Man erhielt 2.0 g (85.8%) 6-Chlor-5-fluor-3-hydroxyindol-2-carbonsäureethylester als braune Kristalle mit einem Smp. von 186-187° (Zers.).

c) Eine Suspension von 2 g (7.7 mmol) 6-Chlor-5-fluor-3-hydroxyindol-2-carbonsäureethylester in 70 ml Methanol wurde mit 50 ml einer 60%igen etherischen Diazomethanlösung versetzt. Nach einer halben Stunde wurden erneut 30 ml dieser Lösung zugegeben und das Gemisch eine weitere halbe Stunde bei Raumtemperatur gerührt. Das Lösungsmittel wurde abdestilliert. Der Rückstand wurde aus Methanol umkristallisiert. Man erhielt 1.34 g (63.6%) 6-Chlor-5-fluor-3-methoxyindol-2-carbonsäureethylester als weisse Kristalle mit Smp. 184-185°.

d) Eine Lösung von 1.33 g (4.9 mmol) 6-Chlor-5-fluor-3-methoxyindol-2-carbonsäureethylester in 24 ml Dibromethan wurde mit 24 ml 28%iger NaOH und 50 mg (0.15 mmol) Tetrabutylammoniumbromid versetzt. Das Gemisch wurde 1.5 Stunden bei 40° gerührt. Die Phasen wurden getrennt und die wässrige Phase wurde mit Toluol extrahiert. Die vereinigten organischen Phasen wurden mit Wasser gewaschen und über Natriumsulfat getrocknet. Man dampfte das Lösungsmittel ab und erhielt 1.85 g (quant.) 1-(2-Bromethyl)-6-chlor-5-fluor-3-methoxyindol-2-carbonsäureethylester als gelbe Kristalle mit Smp. 81.5-82.5°.

e) Eine Suspension von 1.8 g (4.7 mmol) 1-(2-Bromethyl)-6-chlor-5-fluor-3-methoxyindol-2-carbonsäureethylester in 50 ml flüssigem Ammoniak wurde 18 Stunden bei 80° im Autoklaven gerührt. Nach dem Abdampfen des Ammoniaks wurde der Rückstand in Wasser aufgenommen, verrührt und abgenutscht. Man erhielt 1.22 g (96 %) 7-Chlor-8-fluoro-1,2,3,4-tetrahydro-10-methoxy-pyrazino[1,2-a]indol-1-on als beige Kristalle mit Smp. 211-212°.

f) Eine Lösung von 1.2 g (4.4 mmol) 7-Chlor-8-fluor-1,2,3,4-tetrahydro-10-methoxy-pyrazino[1,2-a]indol-1-on in 50 ml trockenem THF wurde mit 0.35 g (9.2 mmol) Lithiumaluminiumhydrid versetzt und 1.5 Stunden unter Rückfluss gekocht. Man zerstörte den Überschuss an Hydrid vorsichtig mit Wasser und gab zu dem Gemisch 6 g Natriumsulfat. Nach Filtieren und Einengen des Filtrats wurde der Rückstand in 50 ml Ethanol gelöst und mit 20 ml gesättigter ethanolischer HCl-Lösung versetzt. Man erhielt 0.75 g (58%) 7-Chlor-8-fluor-1,2,3,4-tetrahydro-10-methoxy-pyrazino[1,2-a]indol-hydrochlorid als weisse Kristalle mit Smp. 224° (Zers.).

Beispiel 15

a) Eine Lösung von 0.48 g (2 mmol) 4-Chlor-5-fluor-indol-2-carbonsäureethylester in 10 ml Dibromethan wurde mit 10 ml 28%iger NaOH und 20 mg (0.06 mmol) Tetrabutylammoniumbromid versetzt. Das Gemisch wurde 2 Stunden bei 50° gerührt. Die Phasen wurden getrennt, und die wässrige Phase wurde mit Toluol extrahiert. Die vereinigten organischen Phasen wurden mit Wasser gewaschen und über Natriumsulfat getrocknet. Das Lösungsmittel wurde abdestilliert, und der feste Rückstand wurde in 20 ml flüssigem Ammoniak suspendiert und 23 Stunden bei 80° im Autoklaven gerührt. Nach dem Abdampfen des Ammoniaks wurde der Rückstand in Wasser aufgenommen, verrührt und abgenutscht. Man erhielt 0.43 g (90%) 9-Chlor-8-fluor-1,2,3,4-tetrahydropyrazino[1,2-a]indol-1-on als beige Kristalle mit einem Smp. von 248-250°.

b) Eine Lösung von 0.42 g (1.7 mmol) 9-Chlor-8-fluor-1,2,3,4-tetrahydro-pyrazino[1,2-a]indol-1-on in 20 ml trockenem THF wurde mit 150 mg (4 mmol) Lithiumaluminiumhydrid versetzt und 2 Stunden unter Rückfluss gekocht. Man zerstörte den Ueberschuss an Hydrid vorsichtig mit Wasser und gab zu dem Gemisch 2 g Natriumsulfat. Nach Filtrieren und Einengen des Filtrats wurde der Rückstand in 20 ml Ethanol gelöst und mit 10 ml gesättigter ethanolischer HCl-Lösung versetzt. Man erhielt 0.2 g (43%) 9-Chlor-8-fluor-1,2,3,4-tetrahydro-pyrazino[1,2-a]indol-hydrochlorid als weisse Kristalle, die sich über 268° zersetzen.

Beispiel A

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| Tablettengewicht | 250 |

Beispiel B

Es werden in üblicher Weise Tabletten folgender Zusammensetzung hergestellt:

| | mg/Tablette |
|---|---|
| Wirkstoff | 200 |
| Lactose pulv. | 100 |
| Maisstärke weiss | 64 |
| Polyvinylpyrrolidon | 12 |
| Na-carboxymethylstärke | 20 |
| Magnesiumstearat | 4 |
| Tablettengewicht | 400 |

Beispiel C

Es werden Kapseln folgender Zusammensetzung hergestellt:

| | mg/Kapsel |
|---|---|
| Wirkstoff | 50 |
| Lactose krist. | 60 |
| Mikrokristalline Cellulose | 34 |
| Talk | 5 |
| Magnesiumstearat | 1 |
| Kapselfüllgewicht | 150 |

Der Wirkstoff mit einer geeigneten Partikelgrösse, die Lactose kristallin und die mikrokristalline Cellulose werden homogen miteinander vermischt, gesiebt und danach Talk und Magnesiumstearat zugemischt. Die Endmischung wird in Hartgelatinekapseln geeigneter Grösse abgefüllt.

**Patentansprüche**

**1.** Verbindungen der allgemeinen Formel

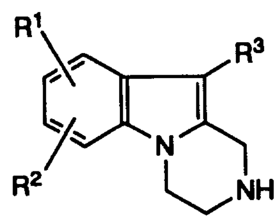

I

worin $R^1$ Wasserstoff, Halogen, Trifluormethyl, niederes Alkyl, Hydroxy oder niederes Alkoxy, $R^2$ Wasserstoff oder Halogen und $R^3$ Wasserstoff, niederes Alkoxy oder niederes Alkylthio bedeuten, mit der Massgabe, dass $R^3$ nur dann Wasserstoff bedeuten kann, wenn $R^1$ und $R^2$ beide von Wasserstoff verschieden sind,
und pharmazeutisch annehmbare Säureadditionssalze der Verbindungen der Formel I.

**2.** Verbindungen nach Anspruch 1, worin $R^3$ von Wasserstoff verschieden ist.

**3.** Verbindungen nach Anspruch 2, worin $R^3$ niederes Alkoxy bedeutet.

**4.** Verbindungen nach einem der Ansprüche 1 bis 3, worin $R^1$ Wasserstoff oder Halogen und $R^2$ Halogen bedeuten.

**5.** Verbindungen nach Anspruch 1, worin $R^3$ Wasserstoff und $R^1$ und $R^2$ je Halogen bedeuten.

**6.** 9-Chlor-8-fluor-1,2,3,4-tetrahydro-10-methoxy-pyrazino[1,2-a]indol.

**7.** 8-Fluor-10-methoxy-1,2,3,4-tetrahydropyrazino[1,2-a]indol.

**8.** 9-Fluor-10-methoxy-1,2,3,4-tetrahydropyrazino[1,2-a]indol.

**9.** 9-Brom-1,2,3,4-tetrahydro-10-methoxy-pyrazino[1,2-a]indol.

**10.** 9-Chlor-8-fluor-1,2,3,4-tetrahydropyrazino[1,2-a]indol.

**11.** 8-Chlor-10-methoxy-1,2,3,4-tetrahydropyrazino[1,2-a]indol;
7-Chlor-1,2,3,4-tetrahydro-10-methoxy-pyrazino[1,2-a]indol;
8-Brom-1,2,3,4-tetrahydro-10-methoxy-pyrazino[1,2-a]indol.

**12.** Verbindungen der allgemeinen Formel

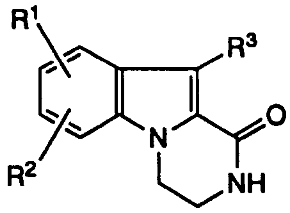

II

worin $R^1$, $R^2$ und $R^3$ die in Anspruch 1 angegebene Bedeutung besitzen.

**13.** Verbindungen nach einem der Ansprüche 1-11 sowie pharmazeutisch annehmbare Säureadditionssalze davon zur Anwendung als therapeutische Wirkstoffe.

**14.** Verbindungen nach einem der Ansprüche 1-11 sowie pharmazeutisch annehmbare Säureadditionssalze davon zur Anwendung als therapeutische Wirkstoff zur Behandlung oder Verhütung von zentralnervösen Störungen, wie Depressionen, bipolare Störungen, Angstzustände, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

**15.** Verfahren zur Herstellung von Verbindungen nach einem der Ansprüche 1-11 und von pharmazeutisch annehmbaren Säureadditionssalzen davon, dadurch gekennzeichnet, dass man eine Verbindung der allgemeinen Formel

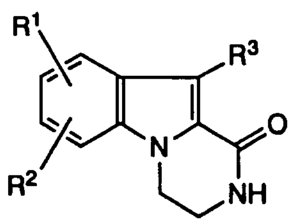

II

worin $R^1$, $R^2$ und $R^3$ die in Anspruch 1 angegebene Bedeutung besitzen,
reduziert und erwünschtenfalls eine erhaltene Verbindung der Formel I in ein pharmazeutisch annehmbares Säureadditionssalz überführt.

**16.** Arzneimittel, enthaltend eine Verbindung nach einem der Ansprüche 1-11 und ein therapeutisch inertes Trägermaterial.

**17.** Arzneimittel nach Anspruch 16 zur Behandlung oder Verhütung von zentralnervösen Störungen, wie Depressionen, bipolare Störungen, Angstzustände, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

**18.** Verwendung von Verbindungen nach einem der Ansprüche 1-11 bei der Behandlung oder Verhütung von Krankheiten.

**19.** Verwendung von Verbindungen nach einem der Ansprüche 1-11 bei der Behandlung oder Verhütung von zentralnervösen Störungen, wie Depressionen, bipolare Störungen, Angstzustände, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung von entsprechenden Arzneimitteln.

Europäisches Patentamt

# EUROPÄISCHER RECHERCHENBERICHT

Nummer der Anmeldung

EP 93 10 8129

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, vol. 113, 1990, Columbus, Ohio, US; abstract no. 6277u, S.B. RAJUR ET AL. 'Synthesis of 1,2,3,4-tetrahydropyrazino[1,2-a]indoles and ethyl 1-(2-aminoethyl)indole-2-carboxylates' Seite 610 ; * Zusammenfassung * & INDIAN JOURNAL OF CHEMISTRY., SECTION B, 1989, 28B(12),1065-68 | 1,16 | C07D487/04 A61K31/495 //(C07D487/04, 241:00,209:00) |
| A | CHEMICAL ABSTRACTS, vol. 78, 1973, Columbus, Ohio, US; abstract no. 29813p, T. OKAMOTO ET AL. 'Piperazinoindole derivatives' Seite 509 ; * Zusammenfassung * & JP-A-7 241 359 (SUMITOMO) 19. Oktober 1992 | 1,17 | |
| A | US-A-3 641 030 (M.E. FREED) 8. Februar 1972 * Spalte 1, Zeile 18 - Zeile 49 * | 1,17 | RECHERCHIERTE SACHGEBIETE (Int. Cl.5) C07D A61K |
| P,A | EP-A-0 521 368 (HOFFMANN-LA ROCHE) 7. Januar 1993 * Ansprüche 1,17 * | 1,17 | |

Der vorliegende Recherchenbericht wurde für alle Patentansprüche erstellt

| Recherchenort | Abschlußdatum der Recherche | Prüfer |
|---|---|---|
| DEN HAAG | 13 SEPTEMBER 1993 | ALFARO FAUS I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0401)